# EUROPEAN PATENT APPLICATION

(11) **EP 3 162 339 A1**
(43) Date of publication of application: **03.05.2017**
(21) Application number: 15191377.9
(22) Date of filing: 26.10.2015
(51) Int. Cl.: A61F 13/49, A61F 13/494, A61F 13/514, A61F 13/537

(54) **ABSORBENT ARTICLE WITH LIQUID-REDIRECTING LAYER**

(71) Applicant: 3M Innovative Properties Company, St. Paul, MN 55133-3427 (US)
(72) Inventor: NEBIGIL, Can, 06550 Ankara (TR)
(74) Representative: Kurz, Arnd

(57) **Abstract**

An absorbent article with a liquid-permeable topsheet, an absorbent core and a liquid-impermeable backsheet is provided with a liquid-redirecting layer. The liquid-redirecting layer may be a water-repellent layer and is located underneath of the absorbent core. A method of manufacturing of an absorbent article having a water-repellent layer underneath of the absorbent core is described.

## Description

### Field

The present disclosure relates to absorbent articles, such as disposable diapers. Furthermore, the present application relates to a method of manufacturing such absorbent articles.

### Background

Absorbent articles including disposable diapers (also called nappies) are widely used to absorb and contain body exudates. They typically contain a top sheet facing the wearer's skin and an absorbent core for receiving and absorbing body exudates. The absorbent core is surrounded by a water impermeable backsheet preventing leakage of exudates to the outside. However, when the absorbent core gets saturated excess liquids may be squeezed out from the absorbent core and may come in contact with the skin where it can cause discomfort and rashes. Solutions have been developed that provide particular multi-layer constructions to either provide additional sealing or absorbing properties. For example, WO2002/36058 A1 discloses an absorbent article where a fluid distribution and storage layer is located below the absorbent core. The additional absorbing layer is meant to absorb fluid that escapes from the absorbent core as a result of pressure being exerted on the core.

However, there is a continuous need for alternative solutions to aid absorption of liquids. In particular there is a need to provide low cost solutions to make such articles affordable also in low income countries.

### Summary

In one aspect there is provided an absorbent article to be worn on the body (1) comprising:
a) a liquid-permeable topsheet (2) having an interior (21) and an exterior surface (22),
b) a liquid-impervious backsheet (3) having an interior (31) and an exterior surface (32),
c) an absorbent core (4) disposed between topsheet (2) and backsheet (3), said core having a surface (42) facing the backsheet (3),
d) a liquid-redirecting layer (5) disposed between absorbent core (4) and backsheet (3) and covering a central area (45) of the surface (42) of the absorbent core facing the backsheet but not covering the periphery (44) of that surface (42), wherein the backsheet (3) and the topsheet (2) are attached to each other.

In another aspect there is provided a method of producing an absorbent article, said method comprises
a) providing:
   (i) a liquid-permeable topsheet (2) having an interior (21) and an exterior surface (22),
   (ii) a liquid-impervious backsheet (3) having an interior (31) and an exterior surface (32),
   (iii) an absorbent core (4) to be disposed between topsheet (2) and backsheet (3), said core having a surface (42) facing the backsheet (3) said surface having a central area (45) and a periphery (44),
   (iv) a liquid-redirecting layer (5) wherein the liquid-redirecting layer (5) is disposed between the absorbent core (4) and the backsheet (3) and covers a central area (45) of the surface (42) of absorbent core but does not cover the periphery (44) of the surface (42),
b) joining topsheet and backsheet.

### Brief description of the drawings

Figure 1 shows a schematic exploded view of an absorbent article according to the present disclosure.
Figure 2 shows a schematic representation of a liquid-redirecting layer according to the present disclosure in the form of a continuous sheet.
Figure 3 shows a schematic representation of an embodiment according to the present disclosure where the liquid-redirecting layer is in the form of a discontinuous layer made up of a series of parallel stripes.
Figure 4 shows another schematic representation of an embodiment of the present disclosure where the liquid-redirecting layer is discontinuous with the discontinuities being cut-out sections.
Figures 5 and 6 show schematic representations of other embodiments according to the present disclosure with types of discontinuous liquid-redirecting layers.

### Detailed Description

All percentages, ratios and proportions used herein are by weight unless otherwise specified.

The present disclosure relates to absorbent articles which absorb and contain body exudates. Such articles are to be worn on the body. They are suitable for use as diapers (also referred to as 'nappies') and adult incontinence products. As used herein, the term "diaper" refers to an absorbent article shaped to be worn on the body by a person or an animal, for example infants or adults. The absorbent articles are placed against or in proximity to the body of the wearer to absorb and contain the various exudates discharged from the body. The absorbent article is typically worn about the lower torso of the wearer and provides a releasable seal to seal off the waist and legs of the wearer from release of liquids. The diapers includes infant diapers, training pants, adult incontinence devices, etc.

Preferably, the absorbent articles are disposable, which means they are not intended to be laundered or otherwise restored or reused as an absorbent article (i.e., they are intended to be discarded after a single use and, preferably, to be recycled, composted or otherwise disposed of in an environmentally compatible manner).

The disposable absorbent articles generally comprise a liquid-pervious first topsheet, a backsheet joined to the topsheet and an absorbent core positioned between the topsheet and the backsheet. Disposable absorbent articles and components thereof, including the topsheet, backsheet, absorbent core, and any individual layers of these components, have an interior surface (or body surface) and an exterior surface (or garment surface). The topsheet as used herein is the sheet that faces the body of the person or animal when being worn while the backsheet as used herein is the sheet that faces away from the body when being worn. As used herein, "interior surface" or "body surface" means that surface of the article or component which is intended to be worn toward or adjacent to the body of the wearer, while the "exterior surface" or "garment surface" is on the opposite side and is intended to be worn toward or placed adjacent to the undergarments when the disposable absorbent article is worn.

The absorbent article according to the present disclosure additionally contains a liquid-redirecting layer.

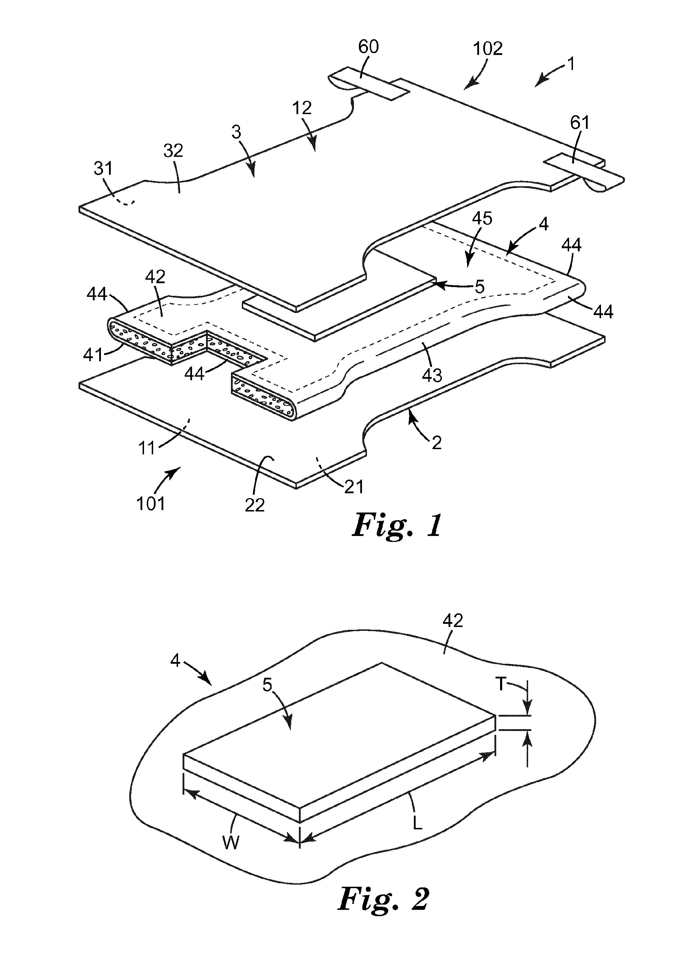

The present disclosure will now be described in greater detail by referring to FIG 1. Fig. 1 shows a schematic exploded view of an absorbent article according to the present disclosure.

As shown in FIG. 1, the absorbent article (1) comprises a liquid-pervious topsheet (2); a liquid-impervious backsheet (3) joined with the topsheet (2) typically along a periphery; an absorbent core (4) positioned between the topsheet (2) and the backsheet (3), and a liquid-redirecting layer (5) between backsheet (3), typically the interior surface (31) of the backsheet (3) and absorbent core (4).

The interior (or body) surface (11) of the absorbent article (1) comprises that portion which is positioned adjacent to the wearer's body during use (i.e., the interior surface generally is formed by at least a portion of the topsheet (2), typically the interior surface (21) of the topsheet (2) - and other components that may be joined to the topsheet (2)). The exterior (or garment) surface (12) comprises that portion of the article (1) which is positioned away from the wearer's body (i.e., the exterior surface (12) generally is formed by at least a portion of the backsheet (3), typically the exterior surface (32) of the backsheet (3) - and other components that may be joined to the backsheet (3).

The absorbent core (4) has an exterior surface (or garment facing surface) (42), and an interior surface (or a body facing surface) (41) and side edges (43). The exterior surface (42) has a periphery (44) which is defined by the lateral edges (43) of the external surface (42). The area at a distance from the periphery (44) forms the central area (45) of the exterior surface (42). Typically, the central area (45) begins at a distance of 0.5 cm from a periphery, more preferably at a distance of 1.0 cm. In some embodiments, the central area begins at a distance of 2.0 cm or 3.0 cm from a periphery. The central area (45) may be symmetrical or asymmetrical. The central area may be continuously at equal distance from the periphery or at unequal distance from the periphery.

A liquid-redirecting layer (5) is positioned on the inner area (45) of the core (4), between the exterior surface (42) of the absorbent core (4) and the body surface of the backsheet (3).

The absorbent article (1) may contain additional components as will be described below, in particular when used as a diaper. Such additional components include, for example, a first cuff and an additional cuff, an elastic waist feature (also referred to as waistband or belt) and a fastening system by which topsheet (2) and backsheet (3) can be releasably joined. In Fig.1 a fastening system (60 and 61) is shown as one type of an additional component.

The individual components of the article (1) will now be described in greater detail

### Absorbent Core (4)

An absorbent core as known in the art may be used in the article of the present disclosure. In general, the absorbent core (4) is capable of absorbing or retaining liquids (e.g., menses, urine, and/or other body exudates). The absorbent core (4) is preferably compressible, conformable, and non-irritating to the wearer's skin. The absorbent core (4) may include any of a wide variety of liquid-absorbent materials commonly used in absorbent articles, such as comminuted wood pulp, which is generally referred to as airfelt. Examples of other suitable absorbent materials for use in the absorbent core include creped cellulose wadding; meltblown polymers; chemically stiffened, modified or cross-linked cellulosic fibers; synthetic fibers such as crimped polyester fibers; peat moss; tissue including tissue wraps and tissue laminates; absorbent foams; absorbent sponges; superabsorbent polymers; absorbent gelling materials; or any equivalent material or combinations of materials, or mixtures of these.
The absorbent core (4) may take on any size or shape that is compatible with the intended use of the article, e.g. a diaper for infants, for animals or for incontinent adults. Exemplary absorbent structures for use as the absorbent core (4) of the present disclosure include those described in U.S. Pat. Application US2005/0177123 A1 and references cited therein (incorporated herein by reference).

The absorbent core (4) can include other absorbent components that are often used in absorbent articles, for example, a dusting layer, a wicking or acquisition layer, or a secondary (e.g., an elasticized) topsheet for increasing the wearer's comfort.

The absorbent core (4) is typically of substantially rectangular shape. It has a central area which is placed at the body where it is most exposed to liquid body exudates. The central area (45) on the surface (42) of the absorbent core that faces the backsheet (3) typically begins at a distance of at least 1 cm, preferably at least 2 cm away from its periphery (44).

### Topsheet (2)

The first topsheet (2) of the abosrbent article (1) is preferably made of a hydrophilic material that promotes rapid transfer of liquids (e.g., urine, menses, and/or runny feces) through the topsheet. Any topsheet as known in the art may be used. If the topsheet (2) is made of a hydrophobic material, at least the upper surface of the topsheet (2) is treated to be hydrophilic so that liquids will transfer through the topsheet more rapidly. This diminishes the likelihood that body exudates will flow off the topsheet (2) rather than being drawn through the topsheet (2) and being absorbed by the absorbent core (4). The topsheet (2) can be rendered hydrophilic by treating it with a surfactant. Suitable methods for treating the topsheet with a surfactant include spraying the topsheet material with the surfactant and immersing the material into the surfactant. A more detailed discussion of such a treatment and hydrophilicity is contained in U.S. Pat. No. 4,988,344 and U.S. Pat. No. 4,988,345. The topsheet (2) is preferably pliant, soft feeling, and non-irritating to the wearer's skin. Further, the topsheet (2) is liquid-pervious, permitting liquids (e.g., menses, urine, and/or runny feces) to readily penetrate through its thickness. A suitable topsheet (2) may be manufactured from a wide range of materials such as woven and nonwoven materials (e.g., a nonwoven web of fibers); polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. When the topsheet (2) comprises a nonwoven web, the web may be manufactured by a wide number of known techniques. For example, the web may be spun-bonded, carded, wet-laid, melt-blown, hydroentangled, combinations of the above, or the like. Additionally, articles according to the present disclosure may comprise topsheets that are elasticized to provide storage compartments for body exudates, particularly bowel movements. Such topsheets and articles containing them are detailed in U.S. Pat. No. 6,482,191.

### Backsheet (3)

The backsheet (3) is impervious to liquids, in particular impervious to low surface tension fluids (e.g., menses, urine, and/or runny feces). The backsheet (3) is preferably manufactured from flexible liquid-impervious materials. As used herein, the term "flexible" refers to materials which are compliant and that readily conform to the general shape and contours of the human body. The backsheet (3) prevents the exudates absorbed and contained in the absorbent core from wetting articles which contact the absorbent article such as bedsheets, pants, pajamas and undergarments. The backsheet (3) may thus comprise a woven or nonwoven material from natural fibers including, for example wood or cotton fibers, synthetic fibers including for example polyolefin fibers and in particular, polypropoylene, polyethylene and polyester fibers. The backsheet material may also comprise polymeric films such as films of polyethylene or polypropylene or polyester or composite materials such as a film-coated nonwoven material. The backsheets (3) may be made by known techniques. For example non-woven backsheets may be made by known web techniques, e. g. spunbonds, carded, wet-laid, melt-blown, hydroentangled and/or combinations of the aforementioned. Typical backsheets are, for example disclosed in US patent application US 2005/0177123 A1.

The backsheet (3) is preferably embossed and/or matte finished and provides a more clothlike-appearance. Further, the backsheet (3) may permit vapors to escape from the absorbent core (i.e., the backsheet is breathable) while still preventing exudates from passing through the backsheet (3).

In a preferred embodiment the backsheet (3) comprises a non-woven material to improve the comfort of the wearer and to increase breathability. The non-woven material may be coated to make more impervious to liquids. In one embodiment the back sheet comprises a non-woven material in its inner surface, i.e., the body surface.

The size of the backsheet (3) is dictated by the size of the absorbent core (4) and the exact absorbent article (1) design selected. A suitable backsheet (3) may have a thickness of from about 0.010 mm to about 5 mm.

### Liquid-redirecting layer (5)

The liquid-redirecting layer (5) is in direct contact with a central area (45) of the absorbent core (4). The size of the liquid-directing layer (5) is smaller than the size of the exterior surface (42) of the core (4) thus ensuring that at least one periphery of the exterior surface (42) is not covered by the liquid-redirecting layer (5). The liquid-redirecting layer (5) does not extend to the periphery of the exterior surface (42) of the core (4). More preferably, the liquid-redirecting layer (5) does not extend beyond the central area (45) of the core (4). The liquid-redirecting layer is of smaller size than the exterior surface (42) to ensure that none of the side edges (43) or at least one of the side edges (43) does not get covered by it. Typically, the liquid-redirecting layer (5) is of at least 5% but less than 90%, preferably less than 75%, of the size of the exterior surface (42).

The liquid-redirecting layer (5) is water-repellent. By contacting and covering the central area of the core, liquid squeezed out from saturated areas of the core will be redirected along the core by the water-repellant surface of the liquid-redirecting layer. When the excess liquids hits areas of the core that are not fully saturated it will be taken up and absorbed by the core. This way the absorbing capacity of the core can be fully exploited.

The liquid-redirecting layer may be water-repellent or has at least a water-repellent surface (being the surface facing the absorbent core). This may be achieved by the liquid-redirecting layer being a single layer made of a water-repellent material. Alternatively the liquid-redirecting layer (5) may be a material that has been coated by a water-repellent material or it may have a water-repellent surface layer otherwise attached or adhered to it (for example by lamination or other forms of adhesion or mechanical attachment like stitching or others).
Water-repellent materials are preferably include materials having a greater contact angel than untreated polypropylene. Typical materials include silicone resins or silicone-resin containing materials, fluoropolymer resin or fluoropolymer resin containing materials. The silicone or fluoropolymer resins may be homopolymers or copolymers of silicone or fluoropolymers with one or more than one other comonomers. Typically, the silicone or fluoropolymer component of in a copolymer makes up at least 25% by weight, preferably at least 45 % by weight or more than 50% by weight based on the total weight of the resin. Suitable silicone resins include those selected from the group consisting polydimethysiloxanes, crosslinked silicones, silicone liquid elastomers, and combinations thereof. Typically, the molecular weight of such silicone polymers should be at least about 4000 MW, preferably at least about 10,000 MW, more preferably at least about 15,000 MW, even more preferably at least about 20,000 MW, and most preferably at least about 25,000 MW. Preferred polydimethylsiloxanes are selected from the group consisting of vinyl-terminated polydimethylsiloxanes, methyl hydrogen dimethylsiloxanes and combinations thereof. Silcione resins and they coating or deposition on substrates are known in the art. Suitable methods and materials include but are not limited to the materials and methods described in European patent application EP 280 36 89 A1. Other methods of preparing multi-layer materials include but are not limited to coextrusion and lamination. Also silicone resin sheets as such may be used.

In one embodiment of the present disclosure, the liquid-redirecting layer may be a single layer prepared from silicone or fluoropolymer resins or a combination thereof. In another embodiment of the present disclosure the liquid-redirecting layer comprises a backing that has been coated with a water-repellent substance such as a silicone-resin or a fluoropolymer resin as described above. The backing may be a woven or non-woven fabric or it may be a continuous or discontinuous, preferably polymeric sheet. Typical materials for a backing include, for example, polyesters, polyethylenes or polypropylenes. In a particular embodiment the liquid-redirecting layer comprises a polyolefin backing, preferably a stretched polyolefin to increase breathability. The backing may further comprise inorganic particles, for example calcium carbonate particle or talcum particles to increase breathability.

Other suitable materials include fluorinated polymers. Suitable fluorinated polymers include polymers containing tetrafluoroethylene and/or perfluorinated or partially fluorinated comonomers. Further suitable materials include tetrafluoroethlene polymers of low molecular weight, referred to in the art as "micro-powder" or soluble partially fluorinated fluoropolymers that may be solvent casted onto a substrate or fluorinated copolymers that can be coextruded onto a substrate. Also fluoropolymer sheets as such may be used.

In a preferred embodiment of the present disclosure the liquid-redirecting layer is a multi-layer article containing in this order a water repellent top layer, a backing and an adhesive layer. The adhesive layer allows the liquid-redirecting layer to be attached to the backsheet. Typical adhesives for attaching components to diaper backsheets as known in the art may be used. Such adhesives include hot melt or pressure sensitive adhesives. Typical adhesives include but are not limited to adhesives based on acrylates.

The liquid-redirecting layer may simply be placed in the absorbent article without any fixation, although fixation, in particular fixation to the backsheet by using adhesives may be advantageous. Other ways of fixation may be used and include, but are not limited to press-fit (between backsheet and absorbent core), releasable mechanical attachments such as hook and loop fastening systems, stitching and lamination.

The liquid-redirecting layer may be a continuous layer and may in its simplest form be a single sheet. Such an embodiment is illustrated in Fig. 2. The sheet has a length L at its longest dimension, a width W at the dimension perpendicular to it and a thickness T. A typical liquid-redirecting layer (5) may have a length of from 3 to 50 cm for a typical infant diaper. It may have a width of from about 1 cm to about 20 cm. Typically, the liquid-redirecting layer (5), irrespective of its shape, may have a thickness of from about 10 microns to 1 cm. The liquid-redirecting layer may typically have a size (length by width in case of a rectangular shape) of 3 to 1000 cm². A typical length or diameter is from about 10 to about 35 cm and a typical width is from about 3 to 10 cm.

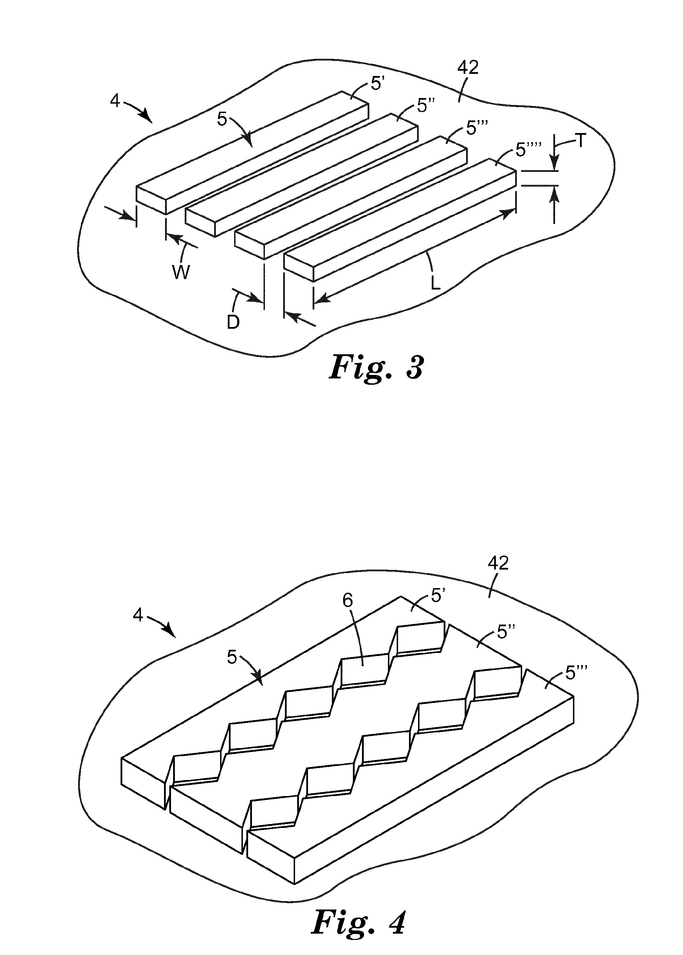

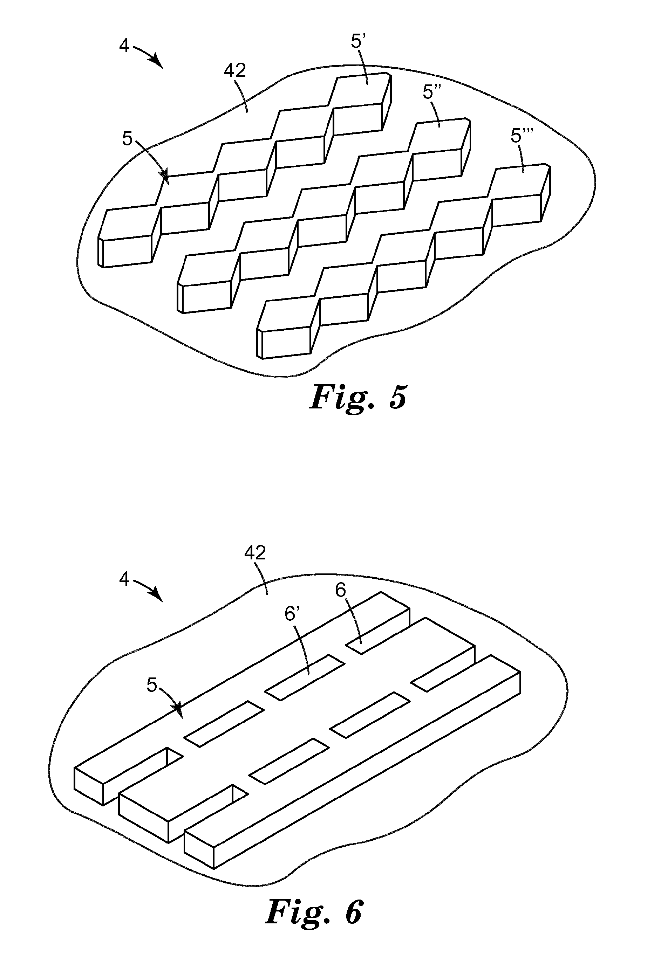

Instead of a single continuous sheet the liquid-redirecting layer may be discontinuous to improve breathability. Some embodiments of discontinuous layers (5) are illustrated in Fig 3 to Fig 6. Discontinuous layers (5) may be composed of a plurality of sheets arranged parallel or not parallel to each other For example a plurality of stripes may be used, for example parallel stripes (5', 5", 5"', 5"") of rectangular extension as illustrated in Fig. 3. The stripes generally may have a length of from about 1cm to about 50 cm and a width of 1 mm to about 20 cm. In some embodiments the distance between the stripes is from 0.1 mm to 10 mm. Other arrangements forming a discontinuous layer include apertures or perforations introduced into the layer as exemplified in Figs 4 and 6. Such apertures and perforations are typically small to still allow the layer to function as a liquid-redirecting layer. For example the total size of the discontinuations or apertures may not make up more than 10% or not more than 20% of the size of the surface of the layer. The discontinuations may from a regular or irregular pattern. Specific embodiments include a liquid-redirecting layer (5) in the form of substantially parallel stripes (5', 5", 5"'), which may have cut-out sections (6) including diamond-shapes cross-sections, as illustrated in Fig. 4.
Another particular embodiment of a liquid-redirecting layer (5) is illustrated in Fig. 6 where the liquid-redirecting layer is a single sheet containing cut-out sections (6, 6'), which in Fig. 6 are illustrated as rectangular cut-outs. The cut-out sections (6, 6') may or may not contact the circumference of the liquid-redirecting layer (5).

The liquid-redirecting layer is dimensioned such that is fits between absorbent core and backsheet. It is smaller in size than the absorbent core and covers the central area of the core which is the area most exposed to body liquids. The liquid-redirecting layer does not extend to the periphery of the absorbent core, or at least one part of the periphery and preferably the entire periphery of the core is not covered by the liquid-redirecting layer. This way it is ensured that the excess liquid is not directed away from the core. The distance between periphery of the core and the liquid-redirecting layer may not be equidistant but may vary and may depend on the shape of the core and the shape of the liquid-redirecting layer. Generally, the liquid-redirecting layer may and periphery of the core may be spaced apart by at least 0.1 mm or at least 1 mm or even at least 10 mm.
The construction of the present disclosure allows to exploit the maximum capacity of the absorbent core by achieving a more homogeneous distribution of liquid along the absorbent core. Therefore, no additional absorbing layer is required and such additional absorbing materials can be spared. In one embodiment of the present disclosure the absorbent article does not contain any second or further absorbent cores. For example, no absorbent core is placed between the liquid-redirecting layer (5) and the backsheet (3).

In one embodiment of the present disclosure the liquid-redirecting layer (5) is a part of the backsheet (3). In this embodiment, the layer (5) is either attached to the backsheet (3) as a separate layer or it is coated onto the backsheet (3), for example as a solvent coating. In this embodiment the layer (5) is positioned and dimensioned such that it does not extend beyond the central area (45) of the core (4).

### Additional Components (not shown in the Figures):

In a preferred embodiment, the absorbent article is a diaper and further comprises one or more of the following: at least a first cuff for providing improved containment of liquids and other body exudates; an elastic waist feature that provides improved fit and containment; and a fastening system which forms a side closure which maintains a first waist region and a second waist region in an overlapping configuration such that lateral tensions are maintained around the circumference of the diaper to maintain the diaper on the wearer. The diaper may also comprise elasticized side panels in the waist regions to provide an elastically extensible feature that provides a more comfortable and contouring fit and more effective application of the diaper.

Cuffs, elastic waist features and fastening systems as known in the art can be used. For example, elasticized leg cuffs can be constructed as described in U.S. Pat. Application US2005/0177123 A1 and references cited therein (incorporated herein by reference). The elasticized waist feature preferably comprises an elasticized waistband (not shown in the figures) that may be constructed in a number of different configurations including those U.S. Pat. Application US2005/0177123 A1 and references cited therein (incorporated herein by reference). This waistband or belt of the diaper or absorbent article may include a cutout of some sort that allows for undisturbed placement of an infant's navel, which tends to be sensitive for a time after birth. Preferably, this cutout is in the shape of a semicircle but may be in any geometric shape that allows for a comfortable fit of the infant's navel. The elasticized side panels may be constructed in a number of configurations as described for example in U.S. Pat. Application US2005/0177123 A1 and references cited therein (incorporated herein by reference).

Known fastening systems for absorbent articles and diapers can be used. Exemplary fastening systems are disclosed in U.S. Pat. Nos. 4,846,815, 4,894,060, 4,946,527, 3,848,594, 4,662,875, and 5,151,092.

The articles of the present disclosure may be prepared by providing the topsheet (2), backsheet (3), absorbent core (4) and liquid-redirecting layer (5).
The liquid-redirecting layer is arranged between absorbent core and backsheet such that it directly contacts the central area (45) of the exterior surface (42) of the core (4). The layer (5) may be attached or coated onto the backsheet before it is placed on its position with respect to the core (4). Layer (5) may also be placed between core (4) and backsheet (3) and will be kept only be joint backsheet and topsheet. After the components have been assembled backsheet and topsheet may be joint together. Additional components may be added after or before backsheet and topsheet are joint.

The backsheet and the topsheet are positioned adjacent the garment surface and the body surface, respectively, of the absorbent core. The absorbent core is preferably joined with the topsheet, the backsheet, or both in any manner as is known by attachment means (not shown in FIG. 1) such as those well known in the art. However, embodiments of the present disclosure are envisioned wherein portions of the entire absorbent core are unattached to one or both of the topsheet and the backsheet. For example, the backsheet and/or the topsheet may be secured to the absorbent core or to each other by a uniform continuous layer of adhesive, a patterned layer of adhesive, or an array of separate lines, spirals, or spots of adhesive. Alternatively, the attachment means may comprise heat bonds, pressure bonds, ultrasonic bonds, dynamic mechanical bonds, or any other suitable attachment means or combinations of these attachment means as are known in the art.

While particular embodiments of the present disclosure have been illustrated and described, it would be obvious to those skilled in the art that various other changes and modifications can be made without departing from the spirit and scope of the invention. It is therefore intended to cover in the appended claims all such changes and modifications that are within the scope of this disclosure.

Reference will now be made in detail to embodiments of the disclosure, one or more examples of which are illustrated in the drawings. Features illustrated or described as part of one embodiment can be used with other embodiments to yield still a third embodiment. It is intended that the present disclosure include these and other modifications and variations.

### EXAMPLES

### Example 1 (comparative)

A diaper (Molfix Baby from Hayat Kimya) was placed with its backsheet on a table and fixed by adhesive tapes in that position. 100 ml of a solution (15%wt of NaCl in distilled water) to which 1 ml of blue food dye had been added were poured over the inner are of the top sheet. A load of 1 kg was placed on the top sheet for one minute. The load was removed. The inner are was colored by the distribution of the test liquid

### Example 2

The test of example 1 was repeated except that the backsheet of the diaper was cut off. A liquid-redirecting layer was placed between core and backsheet covering the central area of the core after which the backsheet was joined again with the topsheet by adhesive tapes. The liquid-redirecting layer was a siliconised adhesive polypropylene sheet having a length of 23 cm, a width of 5 cm and a thickness of 35 microns.

A visual inspection of the area covered by the test liquid showed that the area covered by the test liquid was about 30% greater in example 2 compared to example 1. This demonstrates that the presence of the siliconized layer led to a better distribution of the liquid over the absorbent core. The formation of local oversaturated areas from which liquid can be squeezed out may be avoided or slowed down.

### Example 3 (comparative)

Example 1 was repeated with 60 ml of the test solution used. After the load was removed, a tissue (from Hayat Kimya, Turkey, dimensions 21 cm x 40cm) of known weight was placed on the topsheet for 90 seconds after which it was removed and weighted. The difference in weight indicated the amount of excess liquid absorbed from the tissue.

### Example 4

Example 2 was repeated as described in example 3. A comparison of example 3 and 4 showed that the tissue in example 3 absorbed 34% more liquid than the tissue in example 4. This shows that by using the liquid-redirecting layer the absorbing capacity of the core can be increased.

## Claims

1. An absorbent article to be worn on the body (1) comprising:
a) a liquid-permeable topsheet (2) having an interior (21) and an exterior surface (22),
b) a liquid-impervious backsheet (3) having an interior (31) and an exterior surface (32),
c) an absorbent core (4) disposed between topsheet (2) and backsheet (3), said core having a surface (42) facing the backsheet (3),
d) a liquid-redirecting layer (5) disposed between absorbent core (4) and backsheet (3) and covering a central area (45) of the surface (42) of the absorbent core facing the backsheet but not covering the periphery (44) of that surface (42), wherein the backsheet (3) and the topsheet (2) are attached to each other.

2. The absorbent article (1) of claim 1 wherein the liquid-redirecting layer (5) wherein the central area (45) begins at a distance of at least 1 cm away from the periphery (44) of the surface (42).

3. The absorbent article (1) of any one of the preceding claims wherein the size of the liquid-redirecting layer (5) is at least 10% but less than 90% of the size of the exterior surface (42) of the absorbent core (4).

4. The absorbent article (1) according to any one of the preceding claims wherein the liquid-redirecting layer (5) has a water-repellent surface facing the absorbent core.

5. The absorbent article (1) according to any one of the preceding claims wherein the liquid-redirecting layer (5) contains a coating comprising one or more silicone resins.

6. The absorbent article (1) according to any one of the preceding claims wherein the liquid-redirecting layer (5) comprises a polymeric sheet comprising a polyolefin, wherein the sheet comprises at least at its surface facing the absorbent core (4) one or more silicone resins.

7. The absorbent article (1) according to any one of the preceding claims wherein the liquid-redirecting layer (5) comprises at least one adhesive layer facing the backsheet.

8. The absorbent article (1) according to any one of the preceding claims wherein the liquid-redirecting layer (5) comprises calcium carbonate particles.

9. The absorbent article (1) according to any one of the preceding claims wherein the liquid-redirecting layer (5) is a rectangular stripe.

10. The absorbent article (1) according to any one of the preceding claims wherein no absorbent layer is placed between liquid-redirecting layer (5) and backsheet (3).

11. The absorbent article (1) according to any one of claims 1 to 10 wherein the liquid-redirecting layer (5) is discontinuous.

12. The absorbent article (1) according to any one of claims 1 to 11 wherein the liquid-redirecting layer (5) is discontinuous and comprises a plurality of continuous stripes arranged in parallel.

13. The absorbent article (1) according to any one of claims 1 to 9 wherein the liquid- directing layer (5) s discontinuous and comprises a pattern of perforations.

14. The absorbent article (1) according to any one of the preceding claims being a disposable diaper.

15. A method of producing an absorbent article, said method comprises
c) providing:
(i) a liquid-permeable topsheet (2) having an interior (21) and an exterior surface (22),
(ii) a liquid-impervious backsheet (3) having an interior (31) and an exterior surface (32),
(iii) an absorbent core (4) to be disposed between topsheet (2) and backsheet (3), said core having a surface (42) facing the backsheet (3) said surface having a central area (45) and a periphery (44),
(iv) a liquid-redirecting layer (5) wherein the liquid-redirecting layer (5) is disposed between the absorbent core (4) and the backsheet (3) and covers a central area (45) of the surface (42) of absorbent core but does not cover the periphery (44) of the surface (42),
d) joining topsheet and backsheet.
